# EUROPEAN PATENT APPLICATION

(11) **EP 4 101 440 A1**
(43) Date of publication of application: **14.12.2022**
(21) Application number: 21750964.5
(22) Date of filing: 04.02.2021
(51) Int. Cl.: A61K 9/06, A61K 47/42, A61K 47/02, A61K 9/00, A61P 9/10, A61K 35/34

(54) **COMPOSITION FOR INTRAPERICARDIAL INJECTION COMPRISING STEM CELLS AND USE THEREOF**

(30) Priority: 04.02.2020 KR 20200012963; 03.02.2021 KR 20210015522
(71) Applicant: Hierabio Inc., Seoul 03760 (KR)
(72) Inventor: LEE, Seung Jin, Seoul 07333 (KR); KIM, Heejung, Goyang-si, Gyeonggi-do 10352 (KR); SHIN, Ji Young, Seoul 03902 (KR); JEON, Jeong Hwa, Seoul 03938 (KR)
(74) Representative: Dehns
(86) International application number: PCT/KR2021/001499
(87) International publication number: WO 2021/158045

(57) **Abstract**

The present invention relates to a composition for intrapericardial injection comprising stem cells, and a use thereof, and more particularly, to a composition for improving the therapeutic effect of stem cells by effective transplantation of the stem cells into the pericardium.

## Description

### [Technical Field]

The present invention relates to a composition for intrapericardial injection, which includes stem cells, and a use thereof.

### [Background Art]

Stem cells are cells capable of differentiating into various cells constituting biological tissues, and encompass undifferentiated cells obtained from each tissue of an embryo, a fetus, and an adult before differentiation. Among various types of stem cells, cardiac stem cells are cells residing in the heart and refer to pluripotent stem cells that can differentiate into all cells constituting the heart.

Meanwhile, an ischemic heart disease is a disease caused by insufficient blood supply to a part of the heart muscle due to narrowing of a blood vessel (coronary artery) that supplies blood to the heart, and is one of the diseases whose incidence is significantly increasing due to the radical change in the recent aging society. The ischemic heart disease may be angina pectoris, myocardial infarction and so on, and to treat this, various treatment methods including medication, angioplasty, and heart transplantation have been attempted, but there is still a lack of effective treatment methods for heart disease.

Recently, while, to overcome such a limitation, although research on cell therapy that enables the regeneration of the heart and myocardium using stem cells is being actively conducted, after transplantation of stem cells into the body, not only the differentiation efficiency into cardiac tissue is significantly low, but the survival rate of the transplanted stem cells in the body is low, so cell therapy has limitations in being used as a practical treatment method (Korean Patent No. 10-1915367).

Therefore, if an effective method for promoting angiogenesis as well as increasing the efficiency of differentiation into myocardium using stem cells is developed, it is expected to significantly increase the therapeutic effect on an ischemic heart disease using stem cells.

### [Disclosure]

### [Technical Problem]

To solve the above-described problems of the related art, the present invention is directed to providing a composition for intrapericardial injection, which includes stem cells as an active ingredient to enhance the therapeutic effect of stem cells, and a use thereof.

However, technical problems to be solved in the present invention are not limited to the above-described problems, and other problems which are not described herein will be fully understood by those of ordinary skill in the art from the following descriptions.

### [Technical Solution]

The present invention provides a composition for intrapericardial injection which includes stem cells as an active ingredient.

In one embodiment of the present invention, the stem cells may be stem cell spheroids, which have a diameter of, preferably 10 to 500 µm, and more preferably, 50 to 300 µm or less. In addition, each stem cell spheroid preferably includes, 100 to 5,000 cells, and more preferably, 300 to 3,000 cells.

In another embodiment of the present invention, the composition for intrapericardial injection may further include a hydrogel, which is preferably collagen, gelatin, chondroitin, hyaluronic acid, alginic acid, Matrigel^{™}, chitosan, a peptide, fibrin, polyglycolic acid (PGA), polylactic acid (PLA), polyethylene glycol (PEG), or polyacrylamide, and may be prepared by mixing at least one of the above polymer materials. However, in general, there is no limitation as long as a hydrogel has a form that facilitates intrapericardial injection.

In still another embodiment of the present invention, the fibrin gel may be prepared by mixing thrombin and fibrinogen, and the thrombin is used at a concentration of preferably 0.05 to 5 IU/mL, and more preferably, 0.1 to 2 IU/mL, and the fibrinogen may be used at a concentration of preferably 0.01 to 10 mg/mL, more preferably, 0.05 to 5 mg/mL. There is no limitation on the concentration as long as it can be used to form a fibrin gel. In addition, as the fibrinogen, a human-derived fibrinogen concentrate may also be used.

In yet another embodiment of the present invention, the stem cells are preferably myocardium-derived stem cells (heart stem cells or cardiac progenitor cells), bone marrow-derived stem cells, adipose-derived stem cells, cord blood-derived stem cells, induced pluripotent stem cell, vascular endothelial progenitor cells, hematopoietic stem cells, or neural stem cells, but there is no limitation on the stem cells as long as they can be used for treatment of an ischemic disease.

In addition, the present invention provides a pharmaceutical composition for preventing or treating an ischemic heart disease, which includes the composition for intrapericardial injection as an active ingredient.

In addition, the present invention provides a method of preventing or treating an ischemic disease, which includes administering a composition including the composition for intrapericardial injection as an active ingredient into an individual.

In addition, the present invention provides a use of a composition including the composition for intrapericardial injection as an active ingredient for preventing or treating an ischemic disease.

In addition, the present invention provides a use of the composition for intrapericardial injection for producing a medicine used to treat an ischemic disease.

In one embodiment of the present invention, the ischemic heart disease is preferably myocardial infarction or angina pectoris, but there is no limitation on the ischemic heart disease as long as it is a heart disease that can be treated by the promotion of differentiation into cardiomyocytes or the promotion of cardiovascular formation.

### [Advantageous Effects]

When stem cells are transplanted using a composition for intrapericardial injection according to the present invention, not only the survival rate of an animal model, the degree of stem cell engraftment, and the differentiation of the transplanted stem cells into cardiomyocytes and vascular cells are promoted, but also cardiac tissue damaged by myocardial infarction is effectively regenerated by the transplanted stem cells and blood vessel formation is promoted, thereby significantly improving cardiac function. Therefore, it is expected that stem cells can be effectively used as a therapeutic agent for various ischemic heart diseases using the composition for intrapericardial injection of the present invention.

### [Description of Drawings]

FIG. 1 shows results of confirming the degree of recovery of cardiac function by echocardiography, after transplantation of cardiac stem cells into the myocardium or pericardium in myocardial infarction animal models according to one embodiment of the present invention (^{∗∗} represents p < 0.01).
FIG. 2 shows results of confirming cardiac stem cell spheroids according to one embodiment of the present invention using a microscope. The white bar indicates 100 µm.
FIG. 3 shows a result of confirming the survival rates after transplantation of cardiac stem cell spheroids into myocardial infarction animal models according to one embodiment of the present invention.
FIG. 4 shows a result of confirming, using a fluorescence microscope, whether cardiac stem cell spheroids are normally engrafted into the heart after transplantation of cardiac stem cell spheroids into myocardial infarction animal models according to one embodiment of the present invention.
FIG. 5A shows a result of measuring a cell count according to DiR fluorescence intensity according to one embodiment of the present invention, and FIG. 5B shows a result of confirming the number of cardiac stem cells in the heart according to fluorescence intensity according to one embodiment of the present invention.
FIG. 6 shows results of comparing engraftment rates according to a method of transplanting cardiac stem cell spheroids according to one embodiment of the present invention. The white bar indicates 20 µm and ^{∗} indicates *p* < 0.05.
FIG. 7 shows a result of confirming the differentiation of transplanted heart stem cells according to one embodiment of the present invention by immunohistochemistry. The white bar indicates 20 µm.
FIG. 8 shows results of confirming the effect of regenerating cardiomyocytes by a cardiac stem cell spheroid transplantation method according to one embodiment of the present invention through immunohistochemistry. The white bar indicates 50 µm, ^{∗} indicates that thep value with the Control group is less than 0.05, ^{∗∗} indicates that the *p* value with the Control group is less than 0.01, and # indicates that the *p* value with the CSC spheroid group is less than 0.01.
FIG. 9 shows results of confirming a cardiovascular regeneration effect by a cardiac stem cell spheroid transplantation method according to one embodiment of the present invention through immunohistochemistry. The white bar indicates 50 µm, ^{∗∗} indicates that the *p* value with the Control group is less than 0.01, and # indicates that thep value with the CSC spheroid group is less than 0.01.
FIG. 10 shows a result of confirming the degree of damaged cardiac tissue recovery by a cardiac stem cell spheroid transplantation method according to one embodiment of the present invention through H&E staining. The black bar indicates 50 µm.
FIG. 11 shows results of confirming the degree of damaged cardiac tissue recovery by a cardiac stem cell spheroid transplantation method according to one embodiment of the present invention through MT staining. The black bar indicates 50 µm, ^{∗} indicates that thep value with the Control group is less than 0.05, ^{∗∗} indicates that the *p* value with the Control group is less than 0.01, and # indicates that the *p* value with the CSC spheroid group is less than 0.01.
FIG. 12 shows results of confirming the degree of cardiac function recovery by cardiac stem cell or cardiac stem cell spheroid transplantation according to one embodiment of the present invention through echocardiography. ^{∗∗} indicates p < 0.01, and ^{∗∗∗} indicates p < 0.001.
FIG. 13 shows results of confirming the degree of cardiac function recovery by cardiac stem cell/fibrin gel or cardiac stem cell spheroid/fibrin gel transplantation according to one embodiment of the present invention through echocardiography. ^{∗} and # indicate 0.05, ^{∗∗} and ## indicate 0.01, and ^{∗∗∗} indicates 0.001.

### [Modes of the Invention]

When stem cells are transplanted using a stem cell composition for intrapericardial injection of the present invention, not only the survival rate of an animal model, the degree of stem cell engraftment, and the differentiation of the transplanted stem cells into cardiomyocytes and vascular cells are promoted, but also cardiac tissue damaged by myocardial infarction is effectively regenerated by the transplanted stem cells and blood vessel formation is promoted, thereby significantly improving cardiac function. Therefore, it is expected that stem cells can be effectively used as a cell therapeutic agent for various ischemic heart diseases using the composition for intrapericardial injection of the present invention.

The "stem cell" used herein refers to a broad concept that encompasses undifferentiated cells having the ability to differentiate into various types of body tissue cells, that is, sternness. Such stem cells may be broadly divided into embryonic stem cells, which can be prepared using embryos, adult stem cells, gametes, and cancer stem cells. Among these, cardiac stem cells are stem cells present in the heart, and refer to stem cells with pluripotency, which can differentiate into all cells constituting the heart.

The "spheroid" used herein is the generic term for spherical cell masses produced by 3D culture of cardiac stem cells.

The "ischemic heart disease" used herein is a disease caused by insufficient blood supply to a part of the heart muscle due to narrowing of blood vessels (coronary arteries) that supply blood. This is characterized by an abnormality in the heart due to insufficient blood supply, which prevents the proper supply of oxygen and nutrients necessary for the heart, and types of ischemic heart disease include myocardial infarction and angina pectoris, and specifically, stable angina pectoris, unstable angina pectoris, variant angina pectoris, and acute myocardial infarction.

The "prevention" used herein refers to all actions of inhibiting an ischemic heart disease or delaying the onset thereof by administration of the composition according to the present invention.

The "treatment" used herein refers to all actions involved in alleviating or beneficially changing symptoms of an ischemic heart disease by the administration of the composition according to the present invention.

The "subject" used herein refers to a subject into which the composition of the present invention will be administered, and there is no limitation on the subject.

The "pharmaceutical composition" used herein is prepared in the form of a capsule, a tablet, a granule, an injection, an ointment, a powder, or a drink, and the pharmaceutical composition may target humans. The pharmaceutical composition of the present invention may include a pharmaceutically acceptable carrier. As pharmaceutically acceptable carriers, a binder, a lubricant, a disintegrant, an excipient, a solubilizer, a dispersant, a stabilizer, a suspending agent, a coloring agent and a flavor may be used for oral administration, a mixture of a buffer, a preservative, a pain relief agent, a solubilizer, an isotonic agent and a stabilizer may be used for an injectable, and a base, an excipient, a lubricant and a preservative may be used for local administration. The pharmaceutical composition of the present invention may be prepared in various forms by being mixed with the above-described pharmaceutically acceptable carrier. For example, for oral administration, the pharmaceutical composition of the present invention may be prepared in various dosage forms such as a tablet, a troche, a capsule, an elixir, a suspension, a syrup and a wafer, and for injectables, the pharmaceutical composition of the present invention may be prepared in a unit dose amuple or multiple dose forms. The pharmaceutical composition of the present invention may also be formulated as other forms, such as a dragee, a gel, a pill, a powder, a granule, a suppository, an external preparation, a solution, a suspension, a sustained-release preparation, or a slurry. Meanwhile, examples of carriers, excipients and diluents suitable for formulation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. The examples of carriers, excipients and diluents may also include a filler, an anti-agglomerant, a glidant, a wetting agent, a fragrance, an emulsifier, and a preservative.

Administration routes for the pharmaceutical composition according to the present invention are preferably, but are not limited to, intrapericardial administration, and include, for example, oral, intravenous, intramuscular, intraarticular, intrabursal, intraarterial, intramedullary, intrathecal, intracardiac, transdermal, intracutaneous, subcutaneous, intraperitoneal, intranasal, enteral, topical, sublingual, rectal, intrasternal, intralesional and intracranial administration.

A dose of the pharmaceutical composition of the present invention may vary according to various factors including the activity of a specific compound used, age, body weight, general health, sex, diet, administration time, administration route, excretion rate, drug formulation, and the severity of a specific disease to be prevented or treated, and may be suitably selected by those of ordinary skill in the art depending on a patient's condition, body weight, the severity of a disease, a drug type, an administration route and an administration duration, and may be 0.0001 to 500 mg/kg or 0.001 to 500 mg/kg per day. The pharmaceutical composition of the present invention may be administered once a day or several times in divided portions. The dose does not limit the scope of the present invention in any way.

Hereinafter, to help in understanding the present invention, exemplary examples will be suggested. However, the following examples are merely provided to more easily understand the present invention, and not to limit the present invention.

### [Examples]

### Example 1: Preparation of myocardial infarction animal model and transplantation of cardiac stem cells

The handling and procedures for all animals were performed according to the research protocols approved by the Animal Care and Use Committed of Ewha Womans University (IACUC No: 18-073). To prepare myocardial infarction (MI) animal models, 10- to 12-week-old BALB/c male mice were anesthetized using 3% isoflavone. And then, the heart was checked through a thoracotomy, and the proximal left anterior descending coronary artery was ligated using 7-0 prolene suture (Ethicon^{™}), thereby preparing myocardial infarction animal models.

In addition, to transplant cardiac stem cells, cardiac stem cells were introduced into the animal models by intramyocardial (IM) or pericardial cavity (IP) injection using 27-gauge Hamilton^{®} syringes. More specifically, 5×10⁵ of cardiac stem cells added to 15 µL of a saline solution was introduced by intramyocardial (IM) or intrapericardial injection, and as a control, the same amount of a saline solution was injected.

After the completion of transplantation, the chest and skin were closed, 2 to 5 mg/kg s.c of ketoprofen was given as an analgesic for postoperative pain relief and recovery. In addition, ischemia was identified as the ST segment increased on the electrocardiogram (ECG) from 1 week after transplantation and cardiac cyanosis appeared.

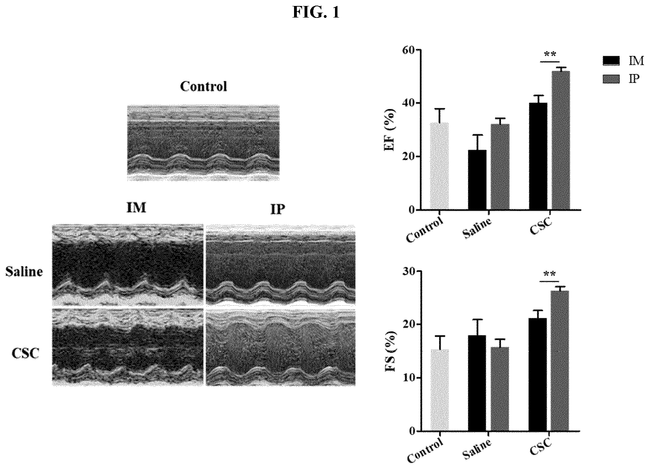

To confirm the therapeutic effect according to the cardiac stem cell transplantation method, cardiac function was accessed by echocardiography. In further detail, the mice were anesthetized two weeks after transplantation and laid on a heating pad at 37 °C, and echocardiography was performed using the VEVO^{®} 2100 micro-ultrasound system (Visual Sonics) equipped with an echocardiography probe (MS-400). The left ventricle (LV) was imaged with both parasternal long-axis and M-mode tracings, and the cine loops of 2D echocardiography were recorded. Ejection fraction (EF) and fractional shortening (FS) values were measured, respectively. The result is shown in FIG. 1.

As shown in FIG. 1, the black bar indicates the result of intramyocardial (IM) injection, and the gray bar indicates the result of intrapericardial injection, and compared with the case of the intramyocardial (IM) injection of the cardiac stem cells, it was confirmed that the intrapericardial injection results in significant recovery of cardiac function, confirming that a myocardial infarction treatment effect can be improved by intrapericardial injection of the cardiac stem cells.

### Example 2: Preparation of cardiac stem cell spheroids

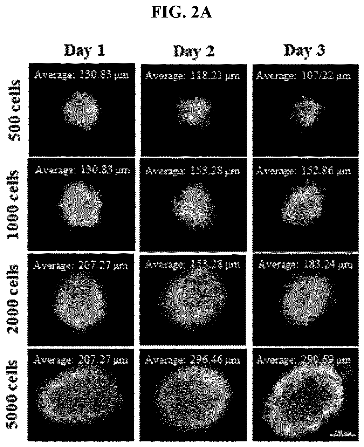

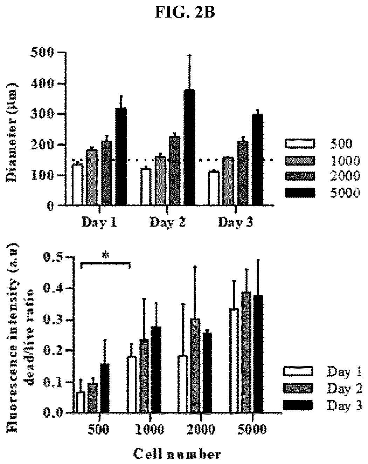

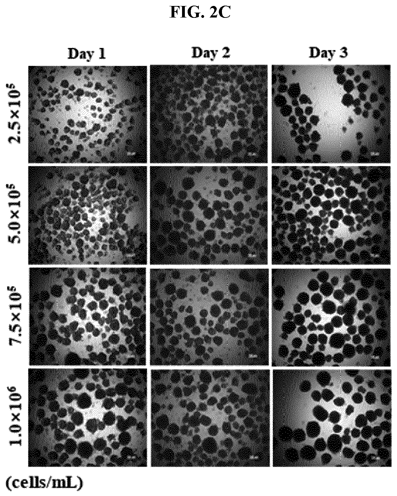

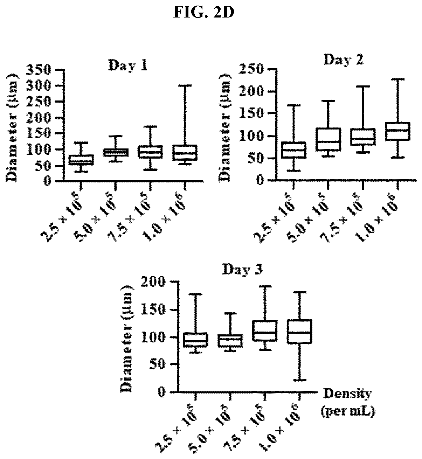

To prepare cardiac stem cell (CSC) spheroids, first, human-derived cardiac stem cells were seeded in a T-75 flask containing a culture medium (DMEM:F-12(1:1 volume ratio) supplemented with 10%(v/v) fetal bovine serum (FBS), 10 ng/ml of an epidermal growth factor (EGF), 2 ng/ml of a basic fibroblast growth factor (bFGF), 10 ng/ml of an insulin-like growth factor-1 (IGF-1) and 1%(v/v) antibiotic-antimycoplasmic reagent (AA)), cultured in a 5% CO₂, 37 °C incubator until confluence reached 80% and then subcultured. The cardiac stem cells cultured until passage 11 were inoculated to have a concentration of 2.5×10⁵, 5×10⁵, 7.5×10⁵, and 1×10⁶ cells/mL into ultra-low attachment surface 6-well culture plates (Corning^{®}) containing DMEM with low glucose, supplemented with 1%(v/v) calf serum (CS), 0.1%(v/v) DMSO, and 50 µg/mL of ascorbic acid and then cultured for three days, thereby preparing cardiac stem cell spheroids. The size of the spheroids formed by each number of cells was represented by measuring the diameter of the prepared spheroid using an optical microscope. The results are shown in FIGS. 2C and 2D. In addition, the formed spheroid was stained using a LIVE/DEAD Viability/Cytotoxicity Assay Kit (Molecular Probes^{™}), and the degree of cell viability according to the number of cells included in the spheroid was confirmed. The results are shown in FIG. 2A and 2B.

As shown in FIGS. 2C and 2D, it was confirmed that the uniform size of cardiac stem cell spheroids with an average diameter of approximately 300 µm were formed. In addition, as shown in FIGS. 2A and 2B, when a spheroid includes 5,000 cells and has a diameter of 500 µm or more, it was confirmed that the death of cells in the spheroid was induced. From the above result, when the spheroids include 200 cells or more and less than 5,000 cells, respectively, or have a diameter of 10 to 500 µm, it was able to be confirmed that they are the most effective cardiac stem cell spheroids that do not induce cell death.

### Example 3: Preparation of composition for intrapericardial injection of spheroids using hydrogel

To prepare a composition for intrapericardial injection using a hydrogel, cardiac stem cell spheroids prepared using 5×10⁵ cells/mL of cardiac stem cells or 5×10⁵ cells/mL of cardiac stem cells were suspended in 7.5 µL of a thrombin solution (0.5 IU/mL), 7.5 µL of a fibrinogen concentrate (0.2 mg/mL) was added to the suspension and reacted, thereby preparing a gel-type composition for intrapericardial injection of cardiac stem cell spheroids.

### Example 4: Transplantation of cardiac stem cells, cardiac stem cell spheroids, cardiac stem cells included in hydrogel, or cardiac stem cell spheroids included in hydrogel

To transplant cardiac stem cell spheroids, first, a myocardial infarction animal model was prepared in the same manner as in Example 1. In addition, 15 µL each of cardiac stem cell spheroids (CSC spheroid) prepared using 5×10⁵ cells/mL of cardiac stem cells, 5×10⁵ cells/mL of cardiac stem cells, cardiac stem cells included in a hydrogel (CSC+gel), or cardiac stem cell spheroids included in a hydrogel (CSC spheroid-Gel), which were prepared in the same manner as in Example 3 was injected into the pericardial cavity of the prepared myocardial infarction animal model using 27-gauge Hamilton^{®} syringes. Hereinafter, in all experiments, the "Sham" group is a group of normal mice that are not treated, the "Control" group is a myocardial infarction animal model, the "Saline" group is a control injected with the same amount of a saline solution, the "CSC" group is an experimental group injected with cardiac stem cells, the "CSC spheroid" group is an experimental group injected with the same amount of cardiac stem cell spheroids, the "CSC+gel" group is an experimental group injected with the same amount of cardiac stem cells included in a hydrogel, and the "CSC spheroid-Gel" group is an experimental group injected with the same amount of cardiac stem cell spheroids included in a hydrogel.

After the completion of transplantation, the chest and skin were closed, 2 to 5 mg/kg s.c of ketoprofen was given as an analgesic for postoperative pain relief and recovery.

### Example 5: Confirmation of therapeutic effect of composition for intrapericardial injection of cardiac stem cell spheroids

### 5.1. Confirmation of survival rate of animal model

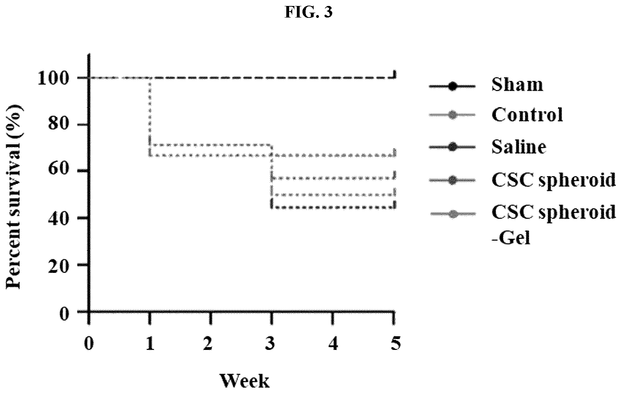

The survival rate of the myocardial infarction animal model transplanted in the same manner as in Example 4 was confirmed with the Kaplan-Meier curve. Afterward, all experiments were repeated at least three times, and the result was expressed as mean ± standard deviation. Statistical significance was confirmed by the Student's t-test between two groups, and among three or more groups, after two-way ANOVA, the Bonferroni's comparison test was used. When *p* < 0.05, it was determined that there is statistical significance. The result is shown in FIG. 3.

As shown in FIG. 3, at week 4, the Control group had a survival rate of approximately 48%, the Saline group had a survival rate approximately 40% lower than the Control group, when the cardiac stem cell spheroids were transplanted, the survival rate was approximately 59%, whereas when the composition for intrapericardial injection of the cardiac stem cell spheroids was transplanted, the survival rate did not decrease from the first week of transplantation and was maintained to be approximately 67% (p<0.01). From the result, it can be confirmed that, as the myocardial infarction treatment effect was exhibited in the case of transplantation of the cardiac stem cell spheroids or the cardiac stem cell spheroids included in a hydrogel, the survival rate reduced by the induction of myocardial infarction (at week 1) was maintained.

### 5.2. Confirmation of engraftment of transplanted cardiac stem cell spheroids

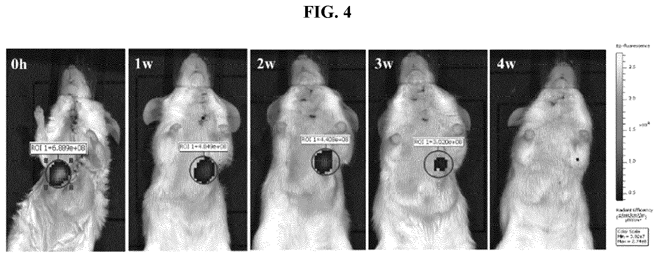

To confirm the ischemic heart disease treatment effect caused by the transplanted cardiac stem cell spheroids, the engraftment of the transplanted cells was examined using DiR. In further detail, the fluorescence of a live animal model was observed using the IVIS^{®}-100 imaging system (Caliper Life Sciences). Fluorescence intensity was calculated as photons/cm² per second. In addition, for cardiac tissue staining, animal models transplanted with cardiac stem cell spheroids were euthanized 4 weeks later, and then cardiac tissues were extracted. In addition, the extracted cardiac tissue was fixed with a 4% paraformaldehyde solution, embedded in paraffin, and cut to a thickness of 0.4 µm to prepare a cardiac tissue section. The results are shown in FIGS. 4 to 6.

FIG. 4 is a set of fluorescence microscopic images confirming the fluorescence of the DiR-labeled cardiac stem cell spheroids, confirming that a fluorescence signal was observed only in the heart, and no fluorescence signals were observed in other tissues such as the lung and liver tissues. From the above result, it was confirmed that the cardiac stem cell spheroids are normally transplanted in the heart.

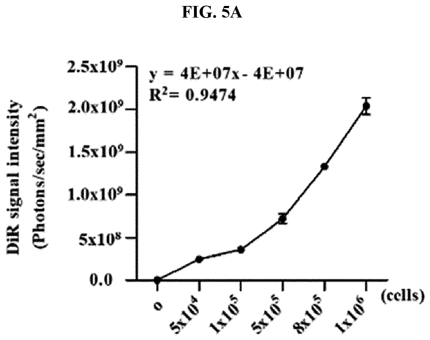

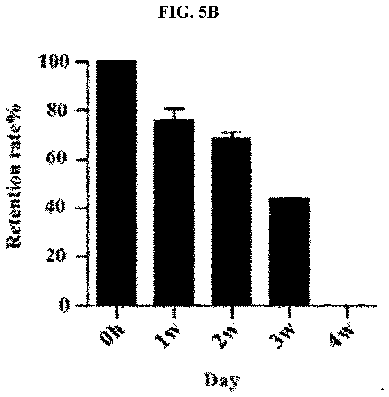

FIG. 5A is a graph of measuring a cell count according to DiR fluorescence intensity, and FIG. 5B shows the retention rate of DiR-labeled cardiac stem cells present in the heart, measured using the fluorescence intensity measured from the heart of an animal model, and confirms that the transplanted DiR-labeled cardiac stem cells gradually decreased and were not observed at week 4 after transplantation.

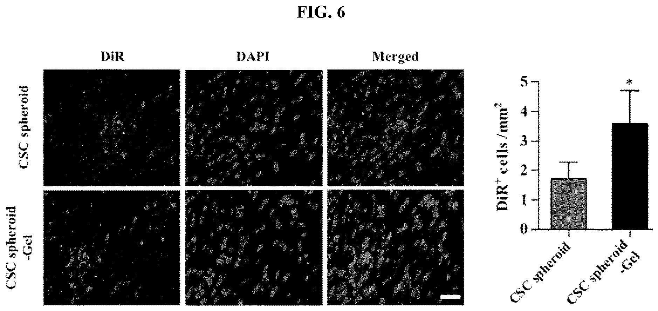

FIG. 6 shows results of staining the cell nuclei in cardiac tissue sections using 4',6-diamidino-2-phenylindole (DAPI) and observing them using a fluorescence microscope. When comparing an animal model directly transplanted with cardiac stem cell spheroids (CSC spheroid) with an animal model transplanted with cardiac stem cell spheroids included in a hydrogel (CSC spheroid-Gel), in both cases of the transplantation of cardiac stem cell spheroids and cardiac stem cell spheroids included in a hydrogel, DiR labeled cardiac stem cells were observed, and therefore, it was confirmed that the transplanted cardiac stem cells survived for 3 weeks or more.

### 5.3. Confirmation of differentiation of transplanted cardiac stem cell spheroids

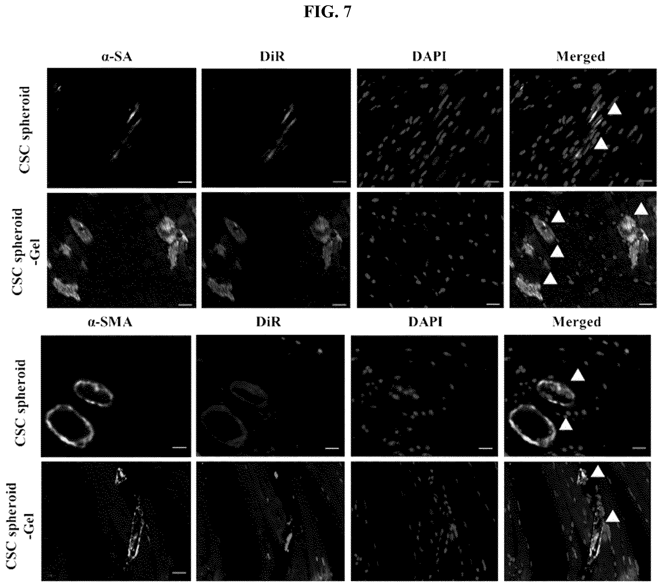

To confirm the ischemic heart disease treatment effect by the transplanted cardiac stem cell spheroids, the cardiovascular differentiation efficiency of the transplanted cells was confirmed. To confirm the cardiovascular differentiation efficiency, the cardiac tissue sections prepared in the same manner as in Example 5.2 were stained with an α-sarcomeric actinin (a-SA) antibody (Abcam) or an α-smooth muscle actin (a-SMA) antibody (Abeam), and DAPI, and observed using a fluorescence microscope. The result is shown in FIG. 7.

As shown in FIG. 7, it was confirmed that α-SA and α-SMA were observed in the same region as the DiR labeled cardiac stem cells. α-SA indicates differentiation into cardiomyocytes, and α-SMA indicates differentiation into vascular smooth muscle cells. From the above result, it can be confirmed that the transplanted cardiac stem cells were stably engrafted in the heart and differentiated into cardiomyocytes and vascular smooth muscle cells.

### 5.4. Confirmation of cardiac regeneration effect by transplantation of cardiac stem cell spheroids

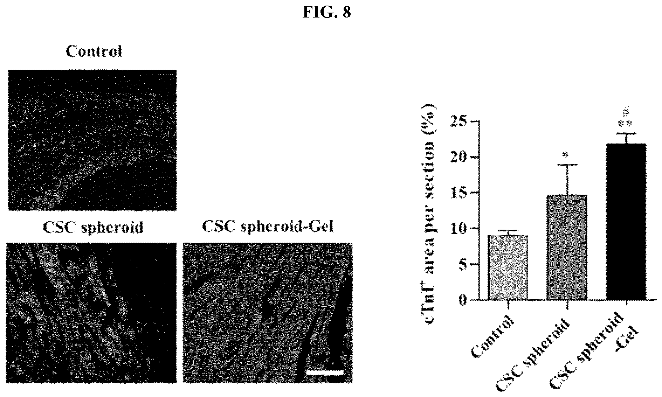

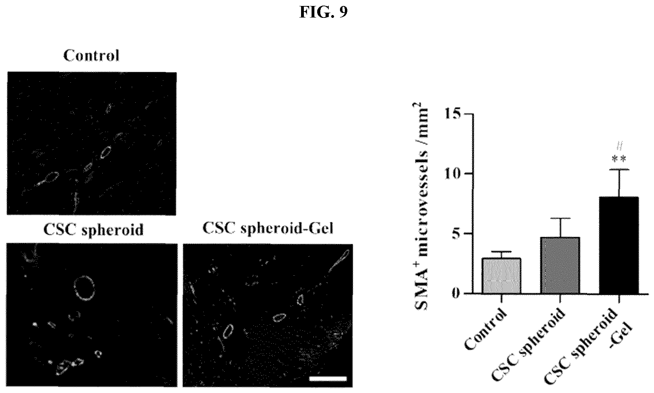

To confirm the cardiac regeneration effect by the transplantation of cardiac stem cell spheroids, cardiac tissue sections prepared in the same manner as in Example 5.2 were stained with a cardiac troponin I (cTnI) antibody (Abeam), an α-SMA antibody (Abeam), and DAPI, and observed using a fluorescence microscope. The results are shown in FIGS. 8 and 9.

As shown in FIG. 8, when the cardiac stem cell spheroids were transplanted by intrapericardial injection, it was confirmed that the area stained with cTnI was significantly increased. Particularly, in the case of the intrapericardial transplantation of cardiac stem cell spheroids included in a hydrogel, it was confirmed that the area stained with cTnI was significantly increased to 21.7%. From the above result, it was confirmed that the regeneration of cardiomyocytes is significantly promoted by the intrapericardial transplantation of cardiac stem cell spheroids, demonstrating that the therapeutic effect on ischemic heart disease can be improved by the promoting the generation of cardiomyocytes.

As shown in FIG. 9, it was confirmed that even when cardiac stem cell spheroids themselves were transplanted, α-SMA positive cells were increased compared with the Control group. However, when cardiac stem cell spheroids included in a hydrogel were intrapericardially injected, α-SMA positive cells significantly increased. From the above result, it was confirmed that the cardiovascular formation can be promoted by intrapericardially injecting the cardiac stem cell spheroids of the present invention, demonstrating that the therapeutic effect on ischemic heart disease can be improved by the promoting the regeneration of heart blood vessels.

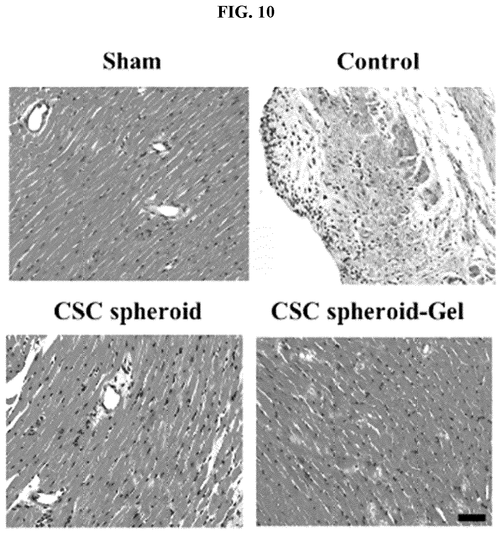

In addition, cardiac tissue sections were stained with hematoxylin-eosin (H&E) stain (Abeam). The result is shown in FIG. 10.

As shown in FIG. 10, it was confirmed that in the case of the Sham group, the cardiac tissue is striped adjacent to the myofibril and shows a branched form of normal cardiac tissue, and in the case of the Control group, myocardial infarction was induced, thereby damaging cardiac tissue, which is not the normal form of cardiac tissue. In addition, inflammatory cells infiltrated, the nuclei aggregated, and necrosis was observed in cardiomyocytes. However, it was confirmed that when cardiac stem cell spheroids or cardiac stem cell spheroids included in a hydrogel were intrapericardially injected, a form of cardiac tissue similar to that of the Sham group was observed. From the above result, it was confirmed that the heart is recovered to a degree similar to that of a normal mouse by the intrapericardial injection of cardiac stem cell spheroids. Moreover, it was confirmed that immune rejection does not occur even without the use of an immunosuppressive drug.

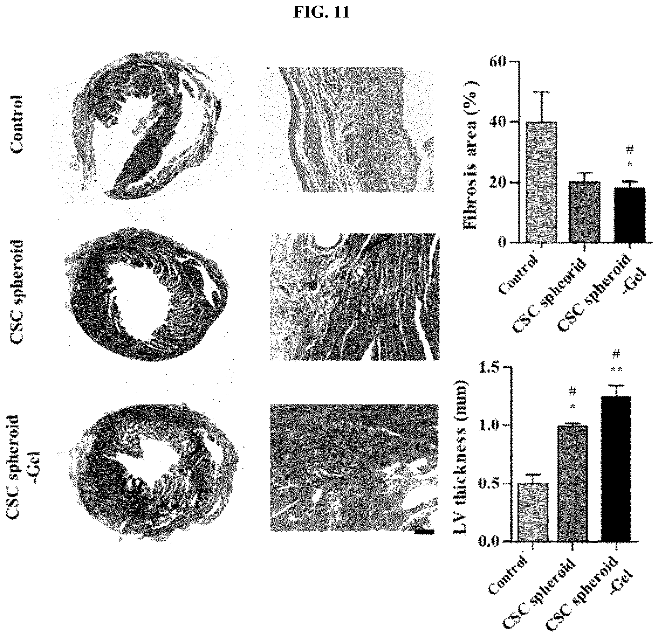

In addition, cardiac tissue sections were stained with Masson's trichrome (MT) stain (Polysciences, Inc.) to quantify a fibrosis level and a left ventricular (LV) wall thickness. In the microscopic image, blue indicates collagen fibers, and red indicates myocytes. The result is shown in FIG. 11.

As shown in FIG. 11, in the case of an animal model in which myocardial infarction was induced, fibrosis was induced around the cardiac tissue and thus the tissue was mostly blue, the LV wall thickness was reduced to approximately 0.5 mm. However, when the cardiac stem cell spheroids themselves were transplanted, it was confirmed that the induction of fibrosis was reduced and the LV wall thickness was increased again. In addition, when cardiac stem cell spheroids were transplanted using a composition for intrapericardial injection of cardiac stem cell spheroids, it was confirmed that the induction of fibrosis was reduced by more than half, and the LV wall thickness was more than doubled to approximately 1.2 mm.

From the above results, when the cardiac stem cell spheroids were transplanted into a myocardial infarction animal model using the composition for intrapericardial injection of cardiac stem cell spheroids of the present invention, the cardiac stem cell spheroids were normally engrafted in the heart not only to stably differentiate into cardiomyocytes or vascular smooth muscle cells, but also to promote the generation of cardiomyocytes and blood vessels even in the surrounding damaged cardiac tissue and effectively regenerate the damaged heart, demonstrating that the therapeutic effect on an ischemic heart disease can be significantly improved.

### 5.5. Confirmation of cardiac function according to transplantation of cardiac stem cells or cardiac stem cell spheroids

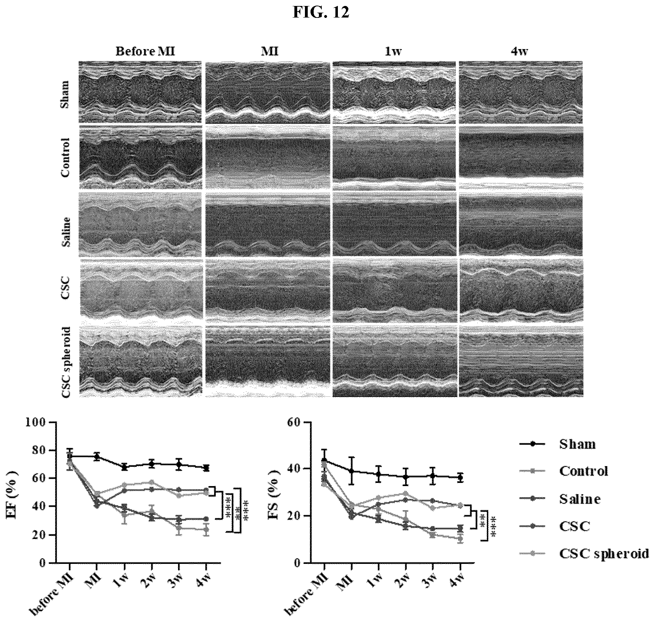

To confirm the therapeutic effect caused by the cardiac stem cell or cardiac stem cell spheroid transplantation, cardiac function was confirmed by echocardiography. In further detail, mice were anesthetized and laid on a heating pad at 37 °C, and echocardiography was performed using a VEVO^{®} 2100 micro-ultrasound system (Visual Sonics) equipped with an echocardiography probe (MS-400). The left ventricle (LV) was imaged with both parasternal long-axis and M-mode tracings, and the cine loops of 2D echocardiography were recorded. An ejection fraction (EF), a fractional shortening (FS), a left ventricle end diastolic diameter (LVEDD), and a left ventricle end systolic diameter (LVESD) were measured, and the measurement was performed before the induction of myocardial infarction, after myocardial infarction, and one week, two weeks, three weeks, and 4 weeks after cardiac stem cell transplantation, respectively. The result is shown in FIG. 12.

As shown in FIG. 12, from the 4^{th} week result, abnormal septal motion was observed due to straightening of a ventricular wall in the Control, Saline and Gel groups, in which myocardial infarction was induced, but in the case of the intrapericardial injection of cardiac stem cells or cardiac stem cell spheroids, it was confirmed that septal motion was restored, and the ejection fraction and fractional shortening, which had been reduced by the induction of myocardial infarction were increased again.

From the above results, it was confirmed that the recovery of cardiac function is significantly increased by transplanting cardiac stem cells or cardiac stem cell spheroids by intrapericardial injection.

### 5.6. Confirmation of cardiac function according to transplantation of cardiac stem cells or cardiac stem cell spheroids included in hydrogel

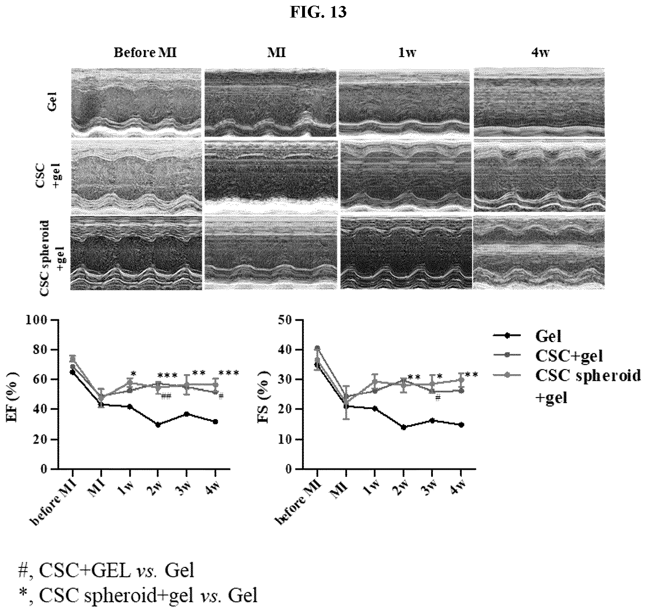

To confirm the therapeutic effect caused by the transplantation of cardiac stem cells or cardiac stem cell spheroids, which is included in a hydrogel, cardiac function was confirmed by echocardiography in the same manner as in Example 5.5. The 'Gel' group is a control injected with only the same amount of a fibrin gel. The result is shown in FIG. 13.

As shown in FIG. 13, from the 4^{th} week result, abnormal septal motion was observed due to straightening of a ventricular wall in the Gel group, but in the case of the intrapericardial injection of cardiac stem cells or cardiac stem cell spheroids, included in a hydrogel, it was confirmed that septal motion was restored to a degree similar to that before myocardial infarction was induced. In addition, it was confirmed that the ejection fraction and fractional shortening that had been reduced by the induction of myocardial infraction are increased again.

From the above results, it was confirmed that, compared to the intramyocardial injection commonly used for stem cell transplantation, intrapericardial injection significantly improved cardiac function, which was lowered by myocardial infarction, confirming that various ischemic heart diseases can be effectively treated by using the composition for intrapericardial injection including cardiac stem cells as an active ingredient according to the present invention. Moreover, by the use of a cardiac stem cell spheroid or hydrogel, not only the survival rate of the animal model, the engraftment of the cardiac stem cell spheroids, and the differentiation of the transplanted cardiac stem cell spheroids into cardiomyocytes and blood vessel cells can be promoted, but also cardiac tissue damaged by myocardial infarction is effectively regenerated by the transplanted cardiac stem cell spheroids and blood vessel formation is promoted, thereby significantly improving cardiac function. Therefore, it was confirmed that various ischemic heart diseases can be effectively treated using the composition for intrapericardial injection of the present invention.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

The composition for intrapericardial injection of stem cells according to the present invention cannot only increase the engraftment rate and differentiation efficiency of stem cells, but also significantly increase the recovery of damaged cardiac tissue and cardiac function by transplanting stem cells through intrapericardial injection to use the stem cells as a therapeutic agent, so that it can be applied to treatment of various ischemic heart diseases and significantly increase therapeutic efficiency.

## Claims

1. A composition for intrapericardial injection, comprising stem cells as an active ingredient.

2. The composition of claim 1, wherein the stem cells are stem cell spheroids.

3. The composition of claim 2, wherein the stem cell spheroid has a diameter of 10 to 500 µm.

4. The composition of claim 2, wherein each stem cell spheroid comprises 100 to 5,000 cells.

5. The composition of claim 1, wherein the composition for intrapericardial injection further comprises a hydrogel.

6. The composition of claim 5, wherein the hydrogel is one or more selected from the group consisting of collagen, gelatin, chondroitin, hyaluronic acid, alginic acid, Matrigel^{™}, chitosan, a peptide, fibrin, polyglycolic acid (PGA), polylactic acid (PLA), polyethylene glycol (PEG), and polyacrylamide.

7. The composition of claim 1, wherein the stem cells are one or more selected from the group consisting of myocardium-derived stem cells, bone marrow-derived stem cells, adipose-derived stem cells, cord blood-derived stem cells, induced pluripotent stem cell, vascular endothelial progenitor cells, hematopoietic stem cells, neural stem cells, and cardiac progenitor cells.

8. A pharmaceutical composition for preventing or treating an ischemic heart disease, comprising: the composition for intrapericardial injection of any one of claims 1 to 7 as an active ingredient.

9. The pharmaceutical composition of claim 8, wherein the ischemic heart disease is myocardial infarction or angina pectoris.

10. A method of preventing or treating an ischemic disease, comprising: administering a composition comprising the composition for intrapericardial injection of any one of claims 1 to 7 as an active ingredient into an individual.

11. A use of a composition comprising the composition for intrapericardial injection of any one of claims 1 to 7 as an active ingredient for preventing or treating an ischemic disease.
